# EUROPÄISCHE PATENTSCHRIFT

(11) **EP 2 287 204 B1**
(45) Veröffentlichungstag und Bekanntmachung des Hinweises auf die Patenterteilung: **30.05.2012**
(21) Anmeldenummer: 10194332.2
(22) Anmeldetag: 20.10.2003
(51) Int. Cl.: C08B 31/18, C08B 30/18, A61K 33/26, A61K 47/48, A61K 31/295

(54) **Wasserlösliche Eisen-Kohlenhydrat-Komplexe, deren Herstellung und diese enthaltende Arzneimittel**
Water-soluble iron-carbohydrate complexes, production thereof, and medicaments containing said complexes
Complexes fer/hydrate de carbone hydrosolubles, leur production et medicaments contenant ces complexes

(30) Priorität: 23.10.2002 DE 10249552
(43) Veröffentlichungstag der Anmeldung: 23.02.2011
(62) Teilanmeldung aus: 03769422.1
(73) Patentinhaber: VIFOR (INTERNATIONAL) AG, 9001 St Gallen (CH)
(72) Erfinder: Geisser, Peter Otto, 9014, St. Gallen (CH); Philipp, Erik, 9303, Wittenbach (CH); Richle, Walter, 9200, Gossau (CH)
(74) Vertreter: Gille Hrabal

(56) Entgegenhaltungen:
- WO-A-02/46241
- CH-A- 423 744
- DE-A1- 3 443 251
- GB-A- 1 111 929
- US-A- 3 086 009
- US-A- 3 821 192

## Beschreibung

Gegenstand der vorliegenden Erfindung sind wasserlösliche Eisen-Kohlenhydrat-Komplexe, die zur Therapie von Eisenmangelanämien geeignet sind, sowie deren Herstellung, diese enthaltende Arzneimittel und deren Verwendung bei der Prophylaxe oder Therapie von Eisenmangelanämien. Die Arzneimittel sind insbesondere zur parenteralen Anwendung geeignet.

Durch Eisenmangel bedingte Anämien können durch Verabreichung von eisenhaltigen Arzneimitteln therapiert oder prophylaktisch behandelt werden. Hierzu ist der Einsatz von Eisen-Kohlenhydrat-Komplexen bekannt. Ein in der Praxis häufig erfolgreich angewandtes Präparat basiert auf einem wasserlöslichen Eisen(III)-Hydroxid-Saccharose-Komplex (Danielson, Salmonson, Derendorf, Geisser, Drug Res., Vol. 46 : 615 - 621, 1996). Im Stand der Technik werden zur parenteralen Verabreichung auch Eisen-Dextran-Komplexe sowie Komplexe auf der Basis schwer zugänglicher Pullulane (WO 02/46241), die unter Druck bei hohen Temperaturen und unter Einbeziehung von Hydrierschritten hergestellt werden müssen beschrieben. Weitere Eisen-Kohlenhydrat-Komplexe sind zur oralen Verabreichung geläufig.

Aus der GB 1111929 ist ein Verfahren zur Herstellung kolloidaler Eisenpräparate bekannt. Aus der DE 3443251 ist ein diagnostisches Mittel zur Anwendung in der NMR-Diagnostik bekannt, welches ferromagnetische Komplexe enthält. Aus der US 3821192 ist ein Verfahren zur Herstellung von Komplexen von Eisen und Dextrin, Maltose oder Glucose bekannt. Aus der US 3086009 ist ein Verfahren zur Herstellung eines Eisenkomplexes mit hydrolisierter Stärke bekannt. Aus der CH 423744 ist ein Verfahren zur Herstellung von Eisenkomplexverbindungen mit oxidierten nicht näher beschriebenen Dextrinen bekannt.

Die vorliegende Erfindung hat sich die Aufgabe gestellt, ein bevorzugt parenteral verabreichbares Eisenpräparat zur Verfügung zu stellen, das sich vergleichsweise einfach sterilisieren lässt; die bisherigen auf Saccharose bzw. Dextran basierenden parenteral verabreichbaren Präparate waren nämlich nur bei Temperaturen bis zu 100°C stabil, wodurch die Sterilisation erschwert wurde. Darüber hinaus soll das erfindungsgemäß bereitzustellende Präparat eine verringerte Toxizität aufweisen und die gefährlichen durch Dextran induzierbaren anaphylaktischen Schocks vermeiden. Auch soll das bereitzustellende Präparat eine hohe Komplexstabilität aufweisen, so dass eine hohe Applikationsdosis bzw, eine hohe Applikationsgeschwindigkeit ermöglicht werden. Auch soll das Eisenpräparat aus einfach erhältlichen Ausgangsprodukten ohne besonderen Aufwand herstellbar sein.

Es hat sich gezeigt, dass diese Aufgabe gelöst wird, durch wasserlösliche Eisen (III)-Kohlenhydrat-Komplexe mit einem gewichtsmittleren Molekulargewicht Mw von 80 kDa bis 400kDa auf der Basis der Oxidationsprodukte von Maltodextrinen. Einen Gegenstand der Erfindung bilden daher wasserlösliche Eisen-Kohlenhydrat-Komplexe, die erhältlich sind aus einer wässrigen Eisen(III)-Salzlösung und einer wässrigen Lösung des Produktes der Oxidation von einem oder mehreren Maltodextrinen mit einer wässrigen Hypochloritlösung bei einem alkalischen pH-Wert von z.B. 8 bis 12, wobei beim Einsatz von einem Maltodetrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose-Äquivalent der am Gemisch beteiligten einzelnen Maltodextrine bei 2 bis 40 liegt.

Einen weiteren Gegenstand der Erfindung bildet ein Verfahren zur Herstellung der erfindungsgemäßen Eisen-Kohlenhydrat-Komplexe, bei dem man ein oder mehrere Maltodextrine in wässriger Lösung bei einem alkalischen pH-Wert von z.B. 8 bis 12 mit einer wässrigen Hypochloritlösung oxidiert und die erhaltene Lösung mit der wässrigen Lösung eines Eisen(III)-Salzes umsetzt, wobei beim Einsatz von einem Maltodextrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose-Äquivalent der am Gemisch beteiligten einzelnen Maltodextrine bei 2 bis 40 liegt,

Die verwendbaren Maltodextrine sind leicht zugängliche Ausgangsprodukte, die im Handel erhältlich sind.

Zur Herstellung der Liganden der erfindungsgemäßen Komplexe werden die Maltodextrine in wässriger Lösung mit Hypochloritlösung oxidiert. Geeignet sind beispielsweise Lösungen von Alkalihypochloriten, wie Natriumhypochloritlösung. Es können handelsübliche Lösungen eingesetzt werden. Die Konzentrationen der Hypochlorit-Lösungen liegen beispielsweise bei mindestens 13 Gew.-%, bevorzugt in der Größenordnung von 13 bis 16 Gew.-% jeweils berechnet als aktives Chlor. Die Lösungen werden bevorzugt in einer derartigen Menge eingesetzt, dass etwa 80 bis 100 %, bevorzugt etwa 90 % einer Aldehydgruppe pro Maltodextrinmolekül oxidiert werden. Auf diese Weise wird das durch die Glucoseanteile der Maltodextrinmoleküle bedingte Reduktionsvermögen auf etwa 20 % oder darunter, bevorzugt 10 % oder darunter verringert.

Die Oxidation erfolgt in alkalischer Lösung, beispielsweise bei pH-Werten von 8 bis 12, z.B. 9 bis 11. Zur Oxidation kann beispielsweise bei Temperaturen in der Größenordnung von 15 bis 40°C, bevorzugt 25 bis 35°C gearbeitet werden. Die Reaktionszeiten liegen beispielsweise in der Größenordnung von 10 Minuten bis 4 Stunden, z.B. 1 bis 1,5 Stunden.

Durch die beschriebene Verfahrensweise wird der Grad der Depolymerisation der eingesetzten Maltodextrine auf einem Minimum gehalten. Ohne eine bindende Theorie abzugeben, wird angenommen, dass die Oxidation vorwiegend an der endständigen Aldehydgruppe (bzw. Acetal- oder Halbacetalgruppe) der Maltodextrinmoleküle erfolgt.

Es ist auch möglich, die Oxidationsreaktion der Maltodextrine zu katalysieren. Geeignet hierzu ist der Zusatz von Bromidionen, z.B. in der Form von Alkalibromiden, beispielsweise Natriumbromid. Die zugesetzte Menge an Bromid ist nicht kritisch. Sie wird möglichst gering gehalten, um ein möglichst leicht zu reinigendes Endprodukt (Fe-Komplex) zu erhalten. Es genügen katalytische Mengen. Wie erwähnt, ist der Zusatz von Bromid zwar möglich, aber nicht erforderlich,

Darüber hinaus ist es beispielsweise auch möglich, das bekannte ternäre Oxidationssystem Hypochlorit/Alkalibomid/2,2,6,6-Tetramethylpiperidin-1-oxyl (TEMPO) zur Oxidation der Maltodextrine zu verwenden. Die Verfahrensweise Maltodextrine unter Katalyse von Alkalibromiden bzw. mit dem ternären TEMPO-System zu oxidieren, wird beispielsweise von Thaburet et al. in Carbohydrate Research 330 (2001) 21 - 29 beschrieben; die dort beschriebene Verfahrensweise ist erfindungsgemäß anwendbar.

Zur Herstellung der erfindungsgemäßen Komplexe werden die erhaltenen oxidierten Maltodextrine in wässriger Lösung mit einem Eisen(III)-Salz umgesetzt. Hierzu können die oxidierten Maltodextrine isoliert und erneut gelöst werden; die erhaltenen wässrigen Lösungen der oxidierten Maltodextrine können jedoch auch direkt zur Weiterverarbeitung mit wässrigen Eisen(III)-Lösungen verwendet werden.

Als Eisen(III)-Salze können wasserlösliche Salze anorganischer oder organischer Säuren oder Mischungen davon verwendet werden, wie Halogenide, z.B. Chlorid und Bromid, oder Sulfate. Bevorzugt werden physiologisch unbedenkliche Salze verwendet. Besonders bevorzugt wird eine wässrige Lösung von Eisen(III)-Chlorid verwendet.

Es hat sich gezeigt, dass sich die Anwesenheit von Chloridionen günstig auf die Komplexbildung auswirkt. Letztere können beispielsweise in der Form von wasserlöslichen Chloriden, wie Alkalimetallchloriden, z.B. Natriumchlorid, Kaliumchlorid oder Ammoniumchlorid, zugesetzt werden. Bevorzugt wird, wie erwähnt, das Eisen(III) in der Form des Chlorids eingesetzt.

Zur Umsetzung kann beispielsweise die wässrige Lösung des oxidierten Maltodextrins mit einer wässrigen Lösung des Eisen(III)-Salzes vermischt werden. Dabei wird bevorzugt so gearbeitet, dass der pH-Wert des Gemisches aus oxidiertem Maltodextrin und Eisen(III)-Salz beim und unmittelbar nach dem Vermischen zunächst stark sauer ist, bzw. so niedrig ist, dass keine Hydrolyse des Eisen(III)-Salzes auftritt, z.B. 2 oder darunter beträgt, um eine unerwünschte Ausfällung von Eisenhydroxiden zu vermeiden, Beim Einsatz von Eisen(III)-Chlorid ist im allgemeinen kein Säurezusatz erforderlich, da wässrige Lösungen von Eisen(III)-Chlorid selbst ausreichend sauer sein können. Nach dem erfolgten Vermischen kann der pH-Wert beispielsweise auf Werte in der Größenordnung von gleich oder größer als 5, beispielsweise bis zu 11, 12, 13 oder 14 angehoben werden. Das Anheben des pH-Wertes erfolgt bevorzugt langsam bzw. allmählich, was beispielsweise dadurch erfolgen kann, dass zunächst eine schwache Base zugesetzt wird, beispielsweise bis zu einem pH von etwa 3; anschließend kann dann mit einer stärkeren Base weiter neutralisiert werden. Als schwache Base kommen beispielsweise Alkali- oder Erdalkalicarbonate, -bicarbonate, wie Natrium- und Kaliumcarbonat oder -bicarbonat oder Ammoniak infrage, Starke Basen sind beispielsweise Alkali- oder Erdalkalihydroxide, wie Natrium-, Kalium-, Calcium- oder Magnesiumhydroxid.

Die Umsetzung kann durch Erwärmen begünstigt werden. Beispielsweise können Temperaturen in der Größenordnung von 15°C bis zur Siedetemperatur angewendet werden. Es ist bevorzugt, die Temperatur allmählich zu steigern. So kann beispielsweise zunächst auf etwa 15 bis 70°C erwärmt und allmählich bis zum Sieden gesteigert werden.

Die Reaktionszeiten liegen beispielsweise in der Größenordnung von 15 Minuten bis zu mehreren Stunden, z.B. 20 Minuten bis 4 Stunden, beispielsweise bei 25 bis 70 Minuten, z.B. 30 bis 60 Minuten.

Die Umsetzung kann im schwach sauren Bereich, beispielsweise bei pH-Werten in der Größenordnung von 5 bis 6, erfolgen. Es hat sich aber gezeigt, dass es zweckmäßig, wenn auch nicht erforderlich ist, den pH-Wert im Verlauf der Komplexbildung auf höhere Werte, bis zu 11, 12, 13 oder 14 anzuheben. Zur Fertigstellung der Reaktion kann der pH-Wert dann durch Säurezusatz weiter gesenkt werden, beispielsweise auf die genannte Größenordnung von 5 bis 6. Als Säuren können anorganische oder organische Säuren oder Gemische davon, insbesondere Halogenwasserstoffsäuren, wie Chlorwasserstoff bzw. wässrige Salzsäure eingesetzt werden.

Wie erwähnt, wird die Komplexbildung im allgemeinen durch Erwärmen begünstigt. Beispielsweise kann bei der bevorzugten Ausführungsform, bei der der pH-Wert im Verlauf der Umsetzung auf Bereiche von über 5 hinaus bis zu 11 oder 14 gesteigert wird, zunächst bei niedrigen Temperaturen in der Größenordnung von 15 bis 70°C, z.B. 40 bis 60°C, z.B. etwa 50°C gearbeitet werden, worauf nach erneuter Verringerung des pH-Wertes beispielsweise auf Werte in der Größenordnung von mindestens 5, allmählich auf Temperaturen über 50°C bis zur Siedetemperatur erwärmt wird.

Die Reaktionszeiten liegen in der Größenordnung von 15 Minuten bis zu mehreren Stunden und können je nach Reaktionstemperatur variieren. Bei der Durchführung des Verfahrens unter zwischenzeitlicher Anwendung von pH-Werten, die über 5 liegen, kann beispielsweise 15 bis 70 Minuten, z.B. 30 bis 60 Minuten bei dem erhöhten pH-Wert, beispielsweise bei Temperaturen bis zu 70°C gearbeitet werden, worauf die Reaktion nach Absenken des pH-Wertes auf den Größenordnungsbereich von mindestens 5, weitere 15 bis 70 Minuten, z.B. 30 bis 60 Minuten bei Temperaturen bis zu beispielsweise 70°C und gegebenenfalls weitere 15 bis 70 Minuten, z.B. 30 bis 60 Minuten bei höheren Temperaturen bis zum Siedepunkt durchgeführt werden kann.

Nach erfolgter Umsetzung kann die erhaltene Lösung beispielsweise auf Raumtemperatur abgekühlt und gegebenenfalls verdünnt und gegebenenfalls filtriert werden. Nach dem Abkühlen kann der pH-Wert durch Zugabe von Säure oder Base auf den Neutralpunkt oder leicht darunter, beispielsweise auf Werte von 5 bis 7 eingestellt werden. Als Säuren oder Basen können beispielsweise die vorstehend zur Umsetzung genannten verwendet werden. Die erhaltenen Lösungen werden gereinigt und können direkt zur Herstellung von Arzneimitteln verwendet werden. Es ist aber auch möglich, die Eisen(III)-Komplexe aus der Lösung zu isolieren, beispielsweise durch Ausfällen mit einem Alkohol, wie einem Alkanol, beispielsweise Ethanol. Die Isolierung kann auch durch Sprühtrocknung erfolgen. Die Reinigung kann in üblicher Weise erfolgen, insbesondere zur Entfernung von Salzen, Dies kann z.B. durch Umkehrosmose erfolgen, wobei eine derartige Umkehrosmose z.B. vor der Sprühtrocknung oder vor dem direkten Einsatz in Arzneimitteln durchgeführt werden kann.

Die erhaltenen Eisen(III)-Kohlenhydrat-Komplexe weisen beispielsweise einen Eisengehalt von 10 bis 40 % Gew./Gew., insbesondere 20 bis 35 % Gew./Gew. auf. Sie sind gut wasserlöslich. Man kann daraus neutrale wässrige Lösungen mit beispielsweise 1 % Gew./Vol. bis 20 % Gew./Vol. Eisengehalt herstellen. Diese Lösungen lassen sich thermisch sterilisieren. Das gewichtsmittlere Molekulargewicht Mw der so erhaltenen Komplexe beträgt 80 kDa bis 400 kDa, bevorzugt 80 bis 350 kDa, besonders bevorzugt bis zu 300 kDa (bestimmt mittels Gelpermeationschromatographie, beispielsweise wie von Geisser et al. in Arzneim. Forsch/Drug Res. 42(II), 12, 1439 - 1452 (1992), Absatz 2.2.5. beschrieben).

Wie erwähnt, lassen sich aus den erfindungsgemäßen Komplexen wässrige Lösungen erstellen. Diese sind insbesondere zur parenteralen Applikation geeignet. Sie können jedoch auch oral oder topisch angewendet werden. Im Gegensatz zu bisher üblichen parenteral verabreichbaren Eisenpräparaten können sie bei hohen Temperaturen, z. B. bei 121°C und darüber sterilisiert werden, bei kurzen Kontaktzeiten von beispielsweise etwa 15 Minuten unter Erreichen von Fₒ ≧ 15. Bei höheren Temperaturen sind die Kontaktzeiten entsprechend kürzer. Bisher bekannte Präparate mussten bei Raumtemperatur steril filtriert und teilweise mit Konservierungsmitteln, wie Benzylalkohol oder Phenol versetzt werden. Derartige Zusätze sind erfindungsgemäß nicht nötig. Es ist möglich, die Lösungen der Komplexe beispielsweise in Ampullen abzufüllen. Beispielsweise lassen sich Lösungen von 1 bis 20 Gew.-%, beispielsweise 5 Gew.-% in Behälter, wie Ampullen oder Stechampullen (Vials) von beispielsweise 2 bis 100 ml, beispielsweise bis zu 50 ml abfüllen. Die Herstellung der parenteral verabreichbaren Lösungen kann in üblicher Weise, gegebenenfalls unter Mitverwendung von für parenterale Lösungen üblichen Zusätzen, erfolgen. Die Lösungen können so formuliert werden, dass sie als solche durch Injektion oder als Infusion, z.B. in Kochsalzlösung, verabreicht werden können. Zur oralen oder topischen Verabreichung können Präparate mit entsprechenden üblichen Exzipienten und Hilfsmitteln formuliert werden.

Einen weiteren Gegenstand der Erfindung bilden daher wässrige Arzneimittel, die insbesondere zur parenteralen, intravenösen, aber auch intramuskulären Verabreichung, sowie zur oralen oder topischen Verabreichung, geeignet sind und insbesondere für die Behandlung von Eisenmangelanämien Verwendung finden können. Ein weiterer Gegenstand der Erfindung betrifft daher auch die Verwendung der erfindungsgemäßen Eisen(III)-Kohlenhydrat-Komplexe zur Behandlung und Prophylaxe von Eisenmangelanämien bzw. zur Herstellung von Arzneimitteln zur insbesondere parenteralen Behandlung von Eisenmangelanämien. Die Arzneimittel sind zum Einsatz in der Human- und der Veterinärmedizin geeignet.

Vorteile, die sich durch die erfindungsgemäßen Eisen-Kohlenhydrat-Komplexe ergeben, sind die bereits vorstehend erwähnten hohen Sterilisierungstemperaturen, die mit einer geringen Toxizität sowie einer verringerten Gefahr anaphylaktischer Schocks einhergehen. Die Toxizität der erfindungsgemäßen Komplexe ist sehr gering. Die LD₅₀ liegt bei über 2000 mg Fe/Kg im Vergleich mit der LD₅₀ der bekannten Pullulankomplexe, die bei 1400 mg Fe/Kg liegt. Durch die große Stabilität der erfindungsgemäß bereitgestellten Komplexe wird es möglich, die Applikationsgeschwindigkeiten sowie auch die Dosierungen zu erhöhen. Auf diese Weise wird es möglich, die erfindungsgemäßen Arzneimittel parenteral als Einmaldosis zu applizieren. Eine derartige Einmaldosis kann beispielsweise 500 bis 1000 mg Eisen betragen; sie kann beispielsweise im Verlauf von 1 Stunde appliziert werden. Ein weiterer Vorteil liegt in der leichten Verfügbarkeit der als Ausgangsprodukte verwendeten Maltodextrine, bei denen es sich z. B. um handelsübliche Zusätze der Nahrungsmittelindustrie handelt.

In der vorliegenden Beschreibung und den nachstehenden Beispielen werden die Dextrose-Äquivalente gravimetrisch bestimmt. Hierzu werden die Maltodextrine in wässriger Lösung mit Fehling'scher Lösung unter Sieden umgesetzt. Die Umsetzung erfolgt quantitativ, d.h. bis keine Entfärbung der Fehling'schen Lösung mehr auftritt. Das ausgefällte Kupfer(I)-Oxid wird bei 105°C bis zur Gewichtskonstanz getrocknet und gravimetrisch bestimmt. Aus den erhaltenen Werten wird der Glucosegehalt (Dextrose-Äquivalent) als % Gew./Gew. der Maltodextrin-Trockensubstanz berechnet. Es kann beispielsweise mit folgenden Lösungen gearbeitet werden: 25 ml Fehling'sche Lösung I, vermischt mit 25 ml Fehling'scher Lösung II; 10 ml wässrige Maltodextrinlösung (10 % Mol/Vol) (Fehling'sche Lösung I: 34,6 g Kupfer(II)-Sulfat gelöst in 500 ml Wasser; Fehling'sche Lösung II: 173 g Kaliumnatriumtartrat und 50 g Natriumhydroxid, gelöst in 400 ml Wasser).

### Beispiel 1

100 g Maltodextrin (9,6 Dextrose-Äquivalente, gravimetrisch bestimmt) werden bei 25°C unter Rühren in 300 ml Wasser gelöst und durch Zugabe von 30 g Natriumhypochloritlösung (13 bis 16 Gew.-% aktives Chlor) bei pH 1 0 oxidiert.

Zu 352 g Eisen(III)-Chloridlösung (12 % Gew./Gew. Fe) werden unter Rühren (Flügelrührer) bei Raumtemperatur zunächst die oxidierte Maltodextrinlösung und dann 554 g Natriumcarbonatlösung (1 7,3 % Gew./Gew.) zugegeben.

Danach wird durch Zugabe von Natronlauge ein pH von 11 eingestellt, die Lösung wird auf 50°C erwärmt und 30 Minuten bei 50°C gehalten. Danach wird durch Zugabe von Salzsäure auf einen pH von 5 bis 6 angesäuert, die Lösung weitere 30 Minuten bei 50°C gehalten und danach auf 97 - 98°C erhitzt und 30 Minuten bei dieser Temperatur gehalten. Nach Abkühlen der Lösung auf Raumtemperatur wird der pH-Wert durch Zusatz von Natronlauge auf 6 - 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 125 g (entsprechend 87 % d. Th.) eines braunen, amorphen Pulvers mit einem Eisengehalt von 29,3 % Gew./Gew. (komplexometfisch ermittelt).

Molekulargewicht Mw 271 kDa

### Beispiel 2

200 g Maltodextrin (9,6 Dextrose-Äquivalente, gravimetrisch bestimmt) werden bei 25°C unter Rühren in 300 ml Wasser gelöst und durch Zugabe von 30 g Natriumhypochloritlösung (13 bis 16 Gew.-% aktives Chlor) bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrodextrinlösung und dann 554 g Natriumcarbonatlösung (17,3 % Gew./Gew.) zugegeben.

Danach wird durch Zugabe von Natronlauge ein pH von 11 eingestellt, die Lösung wird auf 50°C erwärmt und 30 Minuten bei 50°C gehalten. Danach wird durch Zugabe von Salzsäure auf einen pH von 5 bis 6 angesäuert, die Lösung weitere 30 Minuten bei 50°C gehalten und danach auf 97 - 98°C erhitzt und 30 Minuten bei dieser Temperatur gehalten. Nach Abkühlen der Lösung auf Raumtemperatur wird der pH-Wert durch Zusatz von Natronlauge auf 6 - 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 123 g (entsprechend 65 % d. Th.) eines braunen, amorphen Pulvers mit einem Eisengehalt von 22,5 % Gew./Gew. (komplexometrisch ermittelt).

Molekulargewicht Mw 141 kDa

### Beispiel 3

100 g Maltodextrin (9,6 Dextrose-Äquivalente, gravimetrisch bestimmt) werden bei 25°C unter Rühren in 300 ml Wasser gelöst und durch Zugabe von 30 g Natriumhypochloritlösung (13 bis 16 Gew.-% aktives Chlor) und 0,7 g Natriumbromid bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrodextrinlösung und dann 554 g Natriumcarbonatlösung (17,3 % Gew./Gew.) zugegeben.

Danach wird durch Zugabe von Natronlauge ein pH von 6,5 eingestellt, die Lösung wird auf 50°C erwärmt und 60 Minuten bei 50°C gehalten. Danach wird durch Zugabe von Salzsäure auf einen pH von 5 bis 6 angesäuert, die Lösung weitere 30 Minuten bei 50°C gehalten und danach auf 97 - 98°C erhitzt und 30 Minuten bei dieser Temperatur gehalten. Nach Abkühlen der Lösung auf Raumtemperatur wird der pH-Wert durch Zusatz von Natronlauge auf 6 - 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 139 g (entsprechend 88 % d. Th.) eines braunen, amorphen Pulvers mit einem Eisengehalt von 26,8 % Gew./Gew. (komplexometrisch ermittelt).

Molekulargewicht Mw 140 kDa

### Beispiel 4

Eine Mischung aus 45 g Maltodextrin (6,6 Dextrose-Äquivalente, gravimetrisch bestimmt) und 45 g Maltodextrin (14,0 Dextrose-Äquivalente, gravimetrisch bestimmt) wird bei 25°C unter Rühren in 300 ml Wasser gelöst und durch Zugabe von 25 g Natriumhypochloritlösung (13 bis 16 Gew.-% aktives Chlor) und 0,6 g Natriumbromid bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrinlösung und dann 554 g Natriumcarbonatlösung (17,3 % Gew./Gew.) zugegeben.

Danach wird durch Zugabe von Natronlauge ein pH von 11 eingestellt, die Lösung wird auf 50°C erwärmt und 30 Minuten bei 50°C gehalten. Danach wird durch Zugabe von Salzsäure auf einen pH von 5 bis 6 angesäuert, die Lösung weitere 30 Minuten bei 50°C gehalten und danach auf 97 bis 98°C erhitzt und 30 Minuten bei dieser Temperatur gehalten. Nach Abkühlen der Lösung auf Raumtemperatur wird der pH-Wert durch Zusatz von Natronlauge auf 6 bis 7 eingestellt,

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 143 g (entsprechend 90 % d. Th.) eines braunen, amorphen Pulvers mit einem Eisengehalt von 26,5 % Gew./Gew. (komplexometrisch ermittelt).

Molekulargewicht Mw 189 kDa

### Beispiel 5

90 g Maltodextrin (14,0 Dextrose-Äquivalente, gravimetrisch bestimmt) wird bei 25°C unter Rühren in 300 ml Wasser gelöst und durch Zugabe von 35 g Natriumhypochloritlösung (13 bis 16 Gew.-% aktives Chlor) und 0,6 g Natriumbromid bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrinlösung und dann 554 g Natriumcarbonatlösung (17,3 % Gew./Gew.) zugegeben.

Danach wird durch Zugabe von Natronlauge ein pH von 11 eingestellt, die Lösung wird auf 50°C erwärmt und 30 Minuten bei 50°C gehalten, Danach wird durch Zugabe von Salzsäure auf einen pH von 5 bis 6 angesäuert, die Lösung weitere 30 Minuten bei 50°C gehalten und danach auf 97 bis 98°C erhitzt und 30 Minuten bei dieser Temperatur gehalten. Nach Abkühlen der Lösung auf Raumtemperatur wird der pH-Wert durch Zusatz von Natronlauge auf 6 bis 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 131 g (entsprechend 93 % d. Th.) eines braunen, amorphen Pulvers mit einem Eisengehalt von 29,9 % Gew./Gew. (komplexometrisch ermittelt).

Molekulargewicht Mw 118 kDa

### Beispiel 6

Eine Mischung aus 45 g Maltodextrin (5,4 Dextrose-Äquivalente, gravimetrisch bestimmt) und 45 g Maltodextrin (18,1 Dextrose-Äquivalente, gravimetrisch bestimmt) wird bei 25°C unter Rühren in 300 ml Wasser gelöst und durch Zugabe von 31 g Natriumhypochloritlösung (13 bis 16 Gew.-% aktives Chlor) und 0,7 g Natriumbromid bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrinlösung und dann 554 g Natriumcarbonatlösung (17,3 % Gew./Gew.) zugegeben.

Danach wird durch Zugabe von Natronlauge ein pH von 11 eingestellt, die Lösung wird auf 50°C erwärmt und 30 Minuten bei 50°C gehalten. Danach wird durch Zugabe von Salzsäure auf einen pH von 5 bis 6 angesäuert, die Lösung weitere 30 Minuten bei 50°C gehalten und danach auf 97 bis 98°C erhitzt und 30 Minuten bei dieser Temperatur gehalten. Nach Abkühlen der Lösung auf Raumtemperatur wird der pH-Wert durch Zusatz von Natronlauge auf 6 bis 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 134 g (entsprechend 88 % d. Th.) eines braunen, amorphen Pulvers mit einem Eisengehalt von 27,9 % Gew./Gew. (komplexometrisch ermittelt).

Molekulargewicht Mw 1 78 kDa

### Beispiel 7

100 g Maltodextrin (9,6 Dextrose-Äquivalente, gravimetrisch bestimmt) werden bei 25°C unter Rühren in 300 ml Wasser gelöst und durch Zugabe von 29 g Natriumhypochloritlösung (13 bis 16 Gew.-% aktives Chlor) und 0,7 g Natriumbromid bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrinlösung und dann 554 g Natriumcarbonatlösung (17,3 % Gew./Gew.) zugegeben.

Danach wird durch Zugabe von Natronlauge ein pH von 11 eingestellt, die Lösung wird auf 50°C erwärmt und 30 Minuten bei 50°C gehalten. Danach wird durch Zugabe von Salzsäure auf einen pH von 5 bis 6 angesäuert, die Lösung weitere 70 Minuten bei 50°C gehalten. Nach Abkühlen der Lösung auf Raumtemperatur wird der pH-Wert durch Zusatz von Natronlauge auf 6 bis 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 155 g (entsprechend 90 % d. Th.) eines braunen, amorphen Pulvers mit einem Eisengehalt von 24,5 % Gew./Gew. (komplexometrisch ermittelt).

Molekulargewicht Mw 137 kDa

### Beispiel 8

126 g Maltodextrin (6,6 Dextrose-Äquivalente, gravimetrisch bestimmt) werden bei 25°C unter Rühren in 300 ml Wasser gelöst und durch Zugabe von 24 g Natriumhypochloritlösung (1 3 bis 16 Gew.-% aktives Chlor) und 0,7 g Natriumbromid bei pH 10 oxidiert.

Zu 352 g Eisen(III)-Chloridlösung (12 % Gew./Gew. Fe) werden unter Rühren (Flügelrührer) bei Raumtemperatur zunächst die oxidierte Maltrinlösung und dann 554 g Natriumcarbonatlösung (17,3 % Gew./Gew.) zugegeben.

Danach wird durch Zugabe von Natronlauge ein pH von 11 eingestellt, die Lösung wird auf 50°C erwärmt und 30 Minuten bei 50°C gehalten. Danach wird durch Zugabe von Salzsäure auf einen pH von 5 bis 6 angesäuert, die Lösung weitere 70 Minuten bei 50°C gehalten. Nach Abkühlen der Lösung auf Raumtemperatur wird der pH-Wert durch Zusatz von Natronlauge auf 6 bis 7 eingestellt.

Die Lösung wird sodann über einen Sterilfilter filtriert und auf Sedimente geprüft. Danach wird der Komplex durch Ausfällen mit Ethanol im Verhältnis 1 : 0,85 isoliert und im Vakuum bei 50°C getrocknet.

Man erhält 171 g (entsprechend 86 % d. Th.) eines braunen, amorphen Pulvers mit einem Eisengehalt von 21,35 % Gew./Gew. (komplexometrisch ermittelt).

Molekulargewicht Mw 170 kDa

### Vergleich

Im folgenden Vergleich werden die Eigenschaften von erfindungsgemäßen Eisen-Kohlenhydrat-Komplexen einem handelsüblichen Eisen-Saccharose-Komplex gegenübergestellt. Es ist ersichtlich, dass ein erhöhter Eisengehalt möglich ist, eine Thermobehandlung bei höheren Temperaturen durchführbar ist und die Toxizität erfindungsgemäß verringert wird (LD₅₀).

| | erfindungsgemäß | Eisenhydroxid/Saccharose-Komplex |
|---|---|---|
| Fe-Gehalt [%] | 5,0 | 2,0 |
| PH | 5 - 7 | 10,5 - 11,0 |
| Mw [kDa]¹⁾ | 80 - 350 | 34 - 54 |
| Thermobehandlung | 121°C/15' | 100°C/35' |
| LD₅₀ i.v., w.m. [mg Fe/kg Körpergew.] | > 2000 | > 200 |

Bevorzugte Ausführungsformen der Erfindung
1. Wasserlöslicher Eisen-Kohlenhydrat-Komplex, erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose Äquivalent der am Gemisch beteiligten einzelnen Maltodextrine bei 2 bis 40 liegt.
2. Verfahren zur Herstellung eines Eisen-Kohlenhydrat-Komplexes nach Ausführungsform 1, dadurch gekennzeichnet, dass man ein oder mehrere Maltodextrine in wässriger Lösung bei einem alkalischen pH-Wert mit einer wässrigen Hypochloritlösung oxidiert und die erhaltene Lösung mit der wässrigen Lösung eines Eisen(III)-Salzes umsetzt, wobei beim Einsatz von einem Maltodextrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose Äquivalent der am Gemisch beteiligten einzelnen Maltodextrine bei 2 bis 40 liegt.
3. Verfahren nach Ausführungsform 2, dadurch gekennzeichnet, dass die Oxidation des Maltodextrins bzw. der Maltodextrine in Gegenwart von Bromidionen durchgeführt wird.
4. Verfahren nach Ausführungsform 2 oder 3, dadurch gekennzeichnet, dass als Eisen(III)-Salz Eisen(III)-Chlorid verwendet wird.
5. Verfahren nach Ausführungsform 2, 3 oder 4, dadurch gekennzeichnet, dass oxidiertes Maltodextrin und Eisen(III)-Salz zu einer wässrigen Lösung mit einem pH-Wert der so niedrig ist, dass keine Hydrolyse des Eisen(III)-Salzes auftritt gemischt werden, worauf der pH-Wert durch Zusatz von Base auf 5 bis 1 2 angehoben wird.
6. Verfahren nach einer der Ausführungsformen 3 bis 5, dadurch gekennzeichnet, dass man die Umsetzung 15 Minuten bis zu mehreren Stunden bei einer Temperatur von 15°C bis zum Siedepunkt durchführt.
7. Arzneimittel, enthaltend die wässrige Lösung eines Eisen-Kohlenhydrat-Komplexes gemäß Ausführungsform 1 oder 2, oder erhalten gemäß einer der Ausführungsformen 3 bis 6.
8. Arzneimittel gemäß Ausführungsform 7, dadurch gekennzeichnet, dass es zur parenteralen oder oralen Verabreichung formuliert ist.
9. Verwendung der Eisen-Kohlenhydrat-Komplexe von Ausführungsform 1 oder erhalten gemäß einer der Ausführungsformen 2 bis 6, zur Behandlung oder Prophylaxe von Eisenmangelzuständen.
10. Verwendung der Eisen-Kohlenhydrat-Komplexe von Ausführungsform 1 oder erhalten gemäß einer der Ausführungsformen 2 bis 6, zur Herstellung eines Arzneimittels zur Behandlung oder Prophylaxe von Eisenmangelzuständen.
11. Wasserlöslicher Eisen-Kohlenhydrat-Komplex gemäß Ausführungsform 1 für die Behandlung oder Prophylaxe von Eisenmangelzuständen.

## Patentansprüche

1. Wasserlösliche Eisen(III)-Kohlenhydrat-Komplexe auf der Basis der Oxidationsprodukte von Maltodextrinen, worin die Eisen(III)-Kohlenhydrat-Komplexe ein gewichtsmittleres Molekulargewicht Mw von 80 kDa bis 400 kDa aufweisen.

2. Eisen(III)-Kohlenhydrat-Komplexe nach Anspruch 1, worin die Eisen(III)-Kohlenhydrat-Komplexe ein gewichtsmittleres Molekulargewicht Mw von 118 kDa bis 400 kDa aufweisen.

3. Eisen(III)-Kohlenhydrat-Komplexe nach Anspruch 1 oder 2, **dadurch gekennzeichnet, dass** in den Oxidationsprodukten der Maltodextrine 80 bis 100 % der Aldehydgruppen pro Maltodextrinmolekül oxidiert sind.

4. Eisen(III)-Kohlenhydrat-Komplexe nach einem der Ansprüche 1 bis 3, **dadurch gekennzeichnet, dass** in den Oxidationsprodukten der Maltodextrine das durch die Glucoseanteile der Maltodextrinmoleküle bedingte Reduktionsvermögen auf etwa 20 % oder darunter verringert ist.

5. Eisen(III)-Kohlenhydrat-Komplexe nach einem der Ansprüche 1 bis 4, **dadurch gekennzeichnet, dass** in den Oxidationsprodukten der Maltodextrine die Oxidation vorwiegend an der endständigen Aldehydgruppe der Maltodextrinmoleküle erfolgt ist.

6. Eisen(III)-Kohlenhydrat-Komplexe nach einem der Ansprüche 1 bis 5, worin die Oxidationsprodukte der Maltodextrine durch Oxidation von Maltodextrinen mit Hypochloritlösung in wässriger Lösung erhalten werden.

7. Eisen(III)-Kohlenhydrat-Komplexe nach einem der Ansprüche 1 bis 6, worin die Oxidationsprodukte der Maltodextrine durch Oxidation von Maltodextrinen erhalten werden, wobei beim Einsatz von einem Maltodextrin dessen Dextrose-Äquivalent bei 5 bis 20 und beim Einsatz eines Gemisches aus mehreren Maltodextrinen das Dextrose-Äquivalent des Gemisches bei 5 bis 20 und das Dextrose Äquivalent der am Gemisch beteiligten einzelnen Maltodextrine bei 2 bis 40 liegt,

8. Eisen(III)-Kohlenhydrat-Komplexe nach einem der Ansprüche 1 bis 7 zur Verwendung als Arzneimittel zur Behandlung von Eisenmangelanämien.

9. Eisen(III)-Kohlenhydrat-Komplexe nach einem der Ansprüche 1 bis 8 zur Verwendung als Arzneimittel zur parenteralen Verabreichung.

10. Eisen(III)-Kohlenhydrat-Komplexe nach einem der Ansprüche 1 bis 9 zur Verwendung als Arzneimittel zur parenteralen Verabreichung in Form einer Einmaldosis enthaltend 500 bis 1000 mg Eisen.

11. Wässrige Lösungen der Eisen(III)-Kohlenhydrat-Komplexe nach einem der Ansprüche 1 bis 7.

12. Wässrige Lösungen der Eisen(III)-Kohlenhydrat-Komplexe nach Anspruch 11 zur Verwendung als Arzneimittel zur intravenösen Verabreichung.

13. Wässrige Lösung nach Anspruch 11 oder 12, welche einen Eisengehalt von 1 % Gew./Vol. bis 20 % Gew./Vol. aufweist.

14. Wässrige Lösung nach einem der Ansprüche 11 bis 13, welche bei einer Temperatur von 121 °C oder darüber sterilisiert wurde.

15. Wässrige Lösung nach einem der Ansprüche 11 bis 14, welche frei von Konservierungsmitteln ist.

## Claims

1. Water soluble iron(III)-carbohydrate complexes on the basis of the oxidation products of maltodextrins, wherein the iron (III) carbohydrate complexes have a weight average molecular weight M_{w} of 80 kDa to 400 kDa.

2. Iron(III)-carbohydrate complexes according to claim 1, wherein the iron(III)-carbohydrate complexes have a weight average molecular weight M_{w} of 118 kDa to 400 kDa.

3. Iron(III)-carbohydrate complexes according to claims 1 or 2, **characterized in that** in the oxidation products of the maltodextrins 80 to 100 % of the aldehyde groups per molecule are oxidized.

4. Iron(III)-carbohydrate complexes according to any of claims 1 to 3, **characterized in that** in the oxidation of the maltodextrins the reducing power caused by the glucose content of the maltodextrin molecules is lowered to about 20% or less.

5. Iron(III)-carbohydrate complexes according to any of claims 1 to 4, **characterized in that** in the oxidation products of the maltodextrins the oxidation occured predominately at the terminal aldehyde group of the maltodextrin molecules.

6. Iron(III)-carbohydrate complexes according to any of claims 1 to 5, wherein the oxidation of the maltodextrins are obtained by the oxidation of maltodextrins with a hypochlorite solution in an aqueous solution.

7. Iron(III)-carbohydrate complexes according to any of claims 1 to 6, wherein the oxidation products of maltodextrins are obtained by oxidation of maltodextrins, where, when one maltodextrin is applied, its dextrose equivalent Iles between 5 and 20, and when a mixture of several maltodextrins is applied, the dextrose equivalent of the mixture lies between 5 and 20 and the dextrose equivalent of each individual maltodextrin contained in the mixture Iles between 2 and 40.

8. Iron(III)-carbohydrate complexes according to of claims 1 to 7 for use as a medicament for the treatment of iron deficiency anaemia.

9. Iron(III)-carbohydrate complexes according to any of claims 1 to 8 for use as a medicament for parenteral administration.

10. Iron(III)-carbohydrate complexes according to of claims 1 to 9 for use as a medicament for the parenteral administration in the form of a single dose of 500 to 1000 mg iron.

11. Aqueous solutions of the iron(III)-carbohydrate complexes according to any of claims 1 to 7.

12. Aqueous solutions of the iron(III)-carbohydrate complexes according to claim 1 for use as a medicament for the intravenous administration.

13. Aqueous solutions of the iron(III)-carbohydrate complexes according to claims 11 or 12, having an iron content of 1 % weight/vol, to 20% weight/vol.

14. Aqueous solutions of the Iron(III)-carbohydrate complexes according to of claims 11 to 13, which have been sterilized at at a temperature of 121 °C and above.

15. Aqueous solutions of the iron(ill)-carbohydrate complexes according to any of claims 11 to 14, which are devoid of preservatives.

## Revendications

1. Complexes de fer(III)-hydrate de carbone hydrosolubles sur la base des produits de l'oxydation des maltodextrines, dans lesquels les complexes de fer(III)-hydrate de carbone présentent un poids moléculaire moyen en poids M_{w} de 80 kDa à 400 kDa.

2. Complexes de fer(III)-hydrate de carbone selon la revendication 1, dans lesquels les complexes de fer(III)-hydrate de carbone présentent un poids moléculaire moyen en poids M_{w} de 118 kDa à 400 kDa.

3. Complexes de fer(III)-hydrate de carbone selon l'une des revendications 1 ou 2, **caractérisés en ce que** dans les produits de l'oxydation des maltodextrines 80 à 100 % des groupes aldéhyde par molécule de maltodextrine sont oxydés,

4. Complexes de fer(III)-hydrate de carbone selon l'une des revendications 1 à 3, **caractérisés en ce que** dans les produits de l'oxydation des maltodextrines le pouvoir réducteur causé par la teneur en glucose des molécules de maltodextrine est diminué à environ 20% ou inférieur.

5. Complexes de fer(III)-hydrate de carbone selon l'une des revendications 1 à 4, **caractérisés en ce que** dans les produits de l'oxydation des maltodextrines l'oxydation se produit de façon prédominante au groupe aldéhyde terminal des molécules de maltodextrine.

6. Complexes de fer(III)-hydrate de carbone selon l'une des revendications 1 à 5, **caractérisés en ce que** les produits de l'oxydation des maltodextrines sont obtenus par l'oxydation des maltodextrines avec une solution d'hypochlorite dans une solution aqueuse.

7. Complexes de fer(III)-hydrate de carbone selon l'une des revendications 1 à 6, dans lesquels les produits de l'oxydation des maltodextrines sont obtenus par l'oxydation des maltodextrines, dans lesquels, lors de l'utilisation d'une maltodextrine, le Dextrose Équivalent de celle-ci est de 5 à 20 et, lors de l'utilisation d'un de plusieurs maltodextrines, le Dextrose Équivalent de ce mélange est de 5 à 20 et le Dextrose Équivalent de chaque maltodextrine participant as mélange est de 2 à 40.

8. Complexes de fer(III)-hydrate de carbone selon l'une des revendications 1 à 7 pour l'utilisation comme médicament destiné au traitement des anémies dues à une carence de fer.

9. Complexes de fer(III)-hydrate de carbone selon l'une des revendications 1 à 8 pour l'utilisation comme médicament pour une administration par voie parentérale,

10. Complexes de fer(III)-hydrate de carbone selon l'une des revendications 1 à 9 pour l'utilisation comme médicament pour une administration par vole parentérale sous la forme d'une dose unique de 500 à 1000 mg de fer.

11. Solutions aqueuses des complexes de fer(III)-hydrate de carbone selon l'une des revendications 1 à 7.

12. Solutions aqueuses des complexes de fer(III)-hydrate de carbone selon la revendication 11 pour l'utilisation comme médicament pour une administration par voie intraveineuse.

13. Solution aqueuse selon l'une des revendications 11 ou 12, qui présente une teneur en fer de 1 % en poids/en volume à 20 % en poids/en volume.

14. Solution aqueuse selon l'une des revendications 11 à 13, qui a été stérilisée à une température de 121 °C ou supérieur.

15. Solution aqueuse selon l'un des revendications 11 à 14, qui est dénuée des agents de conservation.
